(19) Europäisches Patentamt
European Patent Office
Office européen des brevets

(11) Veröffentlichungsnummer : **0 340 703 B1**

(12) # EUROPÄISCHE PATENTSCHRIFT

(45) Veröffentlichungstag der Patentschrift : **25.03.92 Patentblatt 92/13**

(51) Int. Cl.$^5$ : **C07C 47/293,** C07C 45/65, C07C 47/14, C07C 45/30

(21) Anmeldenummer : **89107877.6**

(22) Anmeldetag : **29.04.89**

(54) **Verfahren zur Herstellung von Formylcyclopropan.**

(30) Priorität : **06.05.88 DE 3815485**

(43) Veröffentlichungstag der Anmeldung : **08.11.89 Patentblatt 89/45**

(45) Bekanntmachung des Hinweises auf die Patenterteilung : **25.03.92 Patentblatt 92/13**

(84) Benannte Vertragsstaaten : **BE CH DE ES FR GB IT LI NL**

(56) Entgegenhaltungen :
**EP-A- 0 219 652**
**US-A- 3 996 259**

(56) Entgegenhaltungen :
**JOURNAL OF ORGANIC CHEMISTRY OF THE USSR, Band 23, Nr. 1, Teil 1, A.KH.KHUSID, "Synthesis of formylcyclopropane, alkyl cyclopropyl ketones, and cyclopropylcarbinols on the basis of 8-elimination under the conditions of phase- transfer catalysis", Seiten 112-115**
**THE JOURNAL OF ORGANIC CHEMISTRY, Band 52, 1987, PIER LUCIO ANELLI et al, "Fast and Selective Oxidation of Primary Alcohols to Aldehydes or to Carboxylic Acids and of Secondary Alcohols to Ketones Mediated by Oxoammonium Salts under Two-Phase Conditions", Seiten 2559-2562**

(73) Patentinhaber : **BASF Aktiengesellschaft**
**Carl-Bosch-Strasse 38**
**W-6700 Ludwigshafen (DE)**

(72) Erfinder : **Kuekenhoehner, Thomas, Dr.**
**Seidelstrasse 2**
**W-6710 Frankenthal (DE)**
Erfinder : **Goetz, Norbert, Dr.**
**Schoefferstrasse 25**
**W-6520 Worms 1 (DE)**

EP 0 340 703 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von Formylcyclopropan (I)

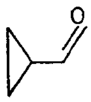

I

durch Cyclisierung von 4-Chlorbutanal bei Temperaturen von 50 - 150°C. Die Erfindung betrifft außerdem eine Ausgestaltung dieses Verfahrens unter Einbeziehung einer besonders vorteilhaften Herstellungsmethode der Ausgangsverbindung 4-Chlorbutanal durch Oxidation von 4-Chlorbutanol.

Formylcyclopropan ist als wichtiger Synthesebaustein zur Einführung der Cyclopropangruppe in chemische Verbindungen von beträchtlichem Interesse, da eine Vielzahl von Pflanzenschutzmitteln, insbesondere Insektizide, Fungizide und Wachstumsregulatoren die Cyclopropylgruppe enthalten.

Formylcyclopropan war bis vor kurzem nur über technisch uninteressante, vielstufige Synthesen erhältlich. Seine Herstellung aus dem leicht zugänglichen und billigen 4-Chlorbutanol durch Oxidation zu 4-Chlorbutanal und dessen anschließende Cyclisierung erschien bis vor kurzem nicht möglich, da Formylcyclopropan im stark basischen wäßrigen Milieu der Cyclisierungsreaktion in einer Cannizzarro-Reaktion zu Hydroxymethylcyclopropan und Cyclopropancarbonsäure disproportioniert (s. hierzu: van der Maeden et al, Rec. Trav. Chim. 91, 221-228 (1972)).

Erst kürzlich gelang Khusid (J. Org. Chem. USSR 23, 112-115 (1987)) eine einfache Synthese von Formylcyclopropan ausgehend von 4-Chlorbutanol. Dabei wird 4-Chlorbutanol mit Pyridinium-Chlorochromat zu 4-Chlorbutanal oxidiert und dieses mit festem Natriumhydroxid in Gegenwart eines wasserunlöslichen organischen Lösungsmittels und eines Phasentransferkatalysators (Fest-Flüssig-Phasentransferkatalyse) zu Formylcyclopropan cyclisiert. Die Anwendung der Fest-Flüssig-Phasentransferkatalyse bei der Cyclisierungsreaktion hat den Zweck, die Cannizzarro-Reaktion des gebildeten Formylcyclopropans, die durch wäßrige Alkalilauge induziert wird, zu verhindern.

Dieses Verfahren liefert im Labormaßstab gute Ausbeuten, ist jedoch für eine übertragung in den industriellen Maßstab ungeeignet, da zum einen bei der Oxidation mit Pyridinium-Chlorochromat große Mengen an Chromsalzen anfallen, welche hohe Entsorgungskosten verursachen, und da zum anderen die Ausführung der Fest-Flüssig-Phasentransferkatalyse im industriellen Maßstab nur sehr schwierig mit aufwendigen technischen Maßnahmen zu bewerkstelligen ist.

Der Erfindung lag daher die Aufgabe zugrunde, ein leicht durchführbares und kostengünstiges Verfahren zu entwickeln, nach welchem Formylcyclopropan aus einfachen Ausgangsverbindungen im industriellen Maßstab - gegebenenfalls kontinuierlich - unter Vermeidung der geschilderten Mängel hergestellt werden kann.

Es wurde nun ein Verfahren zur Herstellung von Formylcyclopropan (I)

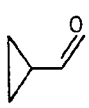

I

durch Cyclisierung von 4-Chlorbutanal mittels einer Mineralbase bei Temperaturen von 50 bis 150°C gefunden, das dadurch gekennzeichnet ist, daß man die Umsetzung mittels einer wäßrigen Mineralbase in einem flüssigen Zweiphasensystem, bestehend aus einer wäßrigen und einer nicht mit Wasser mischbaren, organischen Phase, vornimmt und das Formylcyclopropan laufend aus dem Reaktionsgemisch entfernt.

Außerdem wurde eine Ausgestaltung dieses Verfahrens gefunden, die dadurch gekennzeichnet ist, daß man die Ausgangsverbindung 4-Chlorbutanal durch Oxidation von 4-Chlorbutanol in einem Zweiphasensystem herstellt, wobei als Oxidationsmittel ein System verwendet wird, das aus einer wäßrigen Natriumhypochloritlösung und einer Verbindung der allgemeinen Formel II

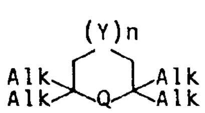

II

besteht, in der Alk gleiche oder verschiedene $C_1$-$C_4$-Alkylgruppen bezeichnet, Q für eine der Gruppierungen

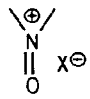 oder 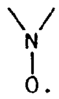 oder 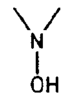

steht, wobei $X^{\ominus}$ ein Anion bedeutet, n 0 oder 1 ist und Y für Sauerstoff, die Carbonylgruppe oder einen Rest

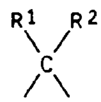

steht, in welchem $R^1$ und $R^2$ Wasserstoff, eine Hydroxylgruppe oder C-organische oder O-organische Reste bedeuten, die auch miteinander zu einem fünf- oder sechsgliedrigen Ring verbunden sein können, und die anschließende Cyclisierung gemäß dem Verfahren nach Anspruch 1 durchführt, ohne das 4-Chlorbutanal vorher zu isolieren.

Die Cyclisierung von 4-Chlorbutanal zu Formylcyclopropan nach Gleichung (1)

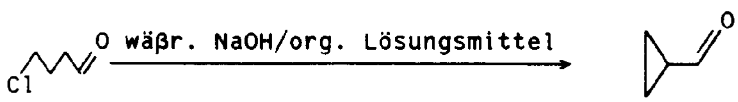

+ NaCl + $H_2O$ (1)

wird in einem Zweiphasensystem, bestehend aus einer wäßrigen und einer nicht mit Wasser mischbaren organischen Phase, durchgeführt. Die Umsetzung kann vorteilhaft in An- als auch in Abwesenheit eines Phasentransferkatalysators durchgeführt werden. Bei beiden Varianten befindet sich die verwendete Base in der wäßrigen Phase, wohingegen das 4-Chlorbutanal in der wasserlöslichen, organischen Phase gelöst ist.

Als organische Lösungsmittel können hierzu beliebige, unter den Reaktionsbedingungen inerte Lösungsmittel wie z.B. Diphenylether, Decalin, Tetralin, Xylol, Diphenyl und Mesitylen eingesetzt werden. Vorzugsweise verwendet man solche Lösungsmittel, deren Siedepunkt höher ist als der Siedepunkt von I oder I enthaltender Azeotrope.

Als wäßrige Mineralbasen verwendet man vorzugsweise wäßrige Lösungen von Alkalimetallhydroxiden, insbesondere solche von Natrium- und Kaliumhydroxid, Alkalimetallcarbonaten, insbesondere solche von Natrium- und Kaliumcarbonat und von Erdalkalimetallhydroxiden, wobei diese Basen bevorzugt als 10 gew.%ige bis gesättigte, besonders bevorzugt 10 bis 25 gew.%ige Lösungen eingesetzt werden.

Als Phasentransferkatalysatoren können prinzipiell beliebige quartäre Ammoniumsalze wie z.B. Methyltributylammoniumbromid, Tetrabutylammoniumchlorid, Methyltrioctylammoniumchlorid; Tetraisopentylammoniumbromid, Decyltributylammoniumbromid, Decyltripropylammoniumperchlorat, Cetyltri butylammoniumhydrogensulfat, Tetraoctylammoniumbenzoat, Cetylpyridiniumchlorid, Triethylbenzylammoniumperfluorooctylsulfonat, Tetrahexylammoniumtosylat in den üblichen katalytischen Mengen zur Anwendung gelangen. Es können ebenso Phosphoniumsalze, z.B. Tetraphenylphosphoniumbromid, Tetrabutylphosphoniumchlorid und Arsoniumsalze wie Tetraphenylarsoniumchlorid als Phasentransferkatalysatoren eingesetzt werden. Natürlich können auch Verbindungen wie Kronenether, Kryptanden oder aliphatische Polyether als solche verwendet werden.

Die Cyclisierung kann bei Temperaturen von 50 bis 150°C, besonders bevorzugt 90 bis 130°C und insbesondere 100 bis 120°C ausgeführt werden. Vorteilhaft wird die Reaktion unter Atmosphärendruck vorgenommen, jedoch ist die Anwendung eines geringen Unterdrucks ebenfalls möglich.

Bei der Durchführung des Verfahrens geht man im allgemeinen so vor, daß man die Base gegebenenfalls zusammen mit dem Phasentransferkatalysator und dem organischen Lösungsmittel vorlegt, das Gemisch auf die Reaktionstemperatur erhitzt und hierzu das 4-Chlorbutanal oder vorteilhaft eine Lösung des 4-Chlorbutanals in einem unter den Reaktionsbedingungen inerten Lösungsmittel zutropft und schon während des Zutropfens das gebildete Formylcyclopropan azeotrop zusammen mit Wasser und Lösungsmittel abdestilliert.

Das 4-Chlorbutanal kann zur Cyclisierung in gereinigter Form verwendet werden. Für die Ausführung des Verfahrens im industriellen Maßstab ist es jedoch besonders günstig, das 4-Chlorbutanal in einer vorgeschalteten Oxidationsreaktion in einem Zweiphasensystem herzustellen und die das 4-Chlorbutanal enthaltende organische Phase direkt, also ohne vorherige Reinigung, zur Cyclisierung einzusetzen. Dieses Vorgehen ist

insofern vorteilhaft, als das 4-Chlorbutanal aufgrund seiner hohen Reaktivität nicht lagerstabil ist, die destillative Aufarbeitung des 4-Chlorbutanals erspart und eine kontinuierliche Verfahrensweise ausgehend von 4-Chlorbutanol ermöglicht wird.

Zur Oxidation des 4-Chlorbutanols ist - bei dieser Ausgestaltung des Verfahrens - die Anwendung der von Anelli et al (J. Org. Chem. 52, 2559-2562 (1987)) eingeführten Oxidationsmethode besonders geeignet. Dabei wird das 4-Chlorbutanol in einem Zweiphasensystem oxidiert, wobei als Oxidationsmittel das System bestehend aus wäßriger Natriumhypochloritlösung, vorzugsweise katalytischen Mengen Kaliumbromid und einer Verbindung der allgemeinen Formel II

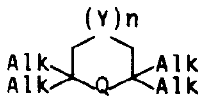

II

dient. Die als eigentliches Oxidationsmittel wirkenden Verbindungen II können als die im Handel erhältlichen Produkte eingesetzt oder aus den entsprechenden Aminen in situ, z.B. durch Umsetzung mit Wasserstoffperoxid, zweckmäßigerweise zusammen mit Natriumwolframat oder ähnlichen metalloxidischen Verbindungen hoher Oxidationsstufe oder besonders vorteilhaft durch Umsetzung mit Peroxycarbonsäuren, insbesondere m-Chlorperoxybenzoesäure, erzeugt werden. Im einzelnen können die Verbindungen II nach Miyazawa et al (J. Org. Chem. 50 1332 (1985)), Briere et al (Bull. Soc.Chim. France 3273 (1965)) und Rozantsev et al (Synthesis 1971, 191) gewonnen werden. 2,2,6,6-Tetramethyl-piperidin-4-on (Triacetonamin) ist ein Handelsprodukt und aus Aceton und Ammoniak zugänglich.

Im einzelnen geht man bei dieser Ausgestaltung des Verfahrens so vor, daß man die Verbindung II in katalytischen Mengen, gelöst in einem organischen Lösungsmittel, vorlegt, anschließend 4-Chlorbutanol, Wasser und vorteilhaft Kaliumbromid hinzufügt und den pH-Wert der wäßrigen Phase mit Hilfe eines Puffers, vorzugsweise eines Phosphat-Puffers, auf einen Wert von 5-9, bevorzugt 6,0 bis 8,0 einstellt. Schließlich wird die Reaktion durch die langsame Zugabe einer 5 bis 20 gew.%igen, vorzugsweise 10 bis 15 gew.%igen, wäßrigen Natriumhypochloritlösung bei 0 bis 40°C, bevorzugt bei Raumtemperatur, gestartet.

Als organische Lösungsmittel sind alle solchen geeignet, welche nicht mit Wasser mischbar sind und sich unter den Bedingungen der Oxidationsreaktion sowie der nachfolgenden Cyclisierungsreaktion inert verhalten. Beispielsweise seien Dichlormethan, Dichlorethan, Tetrachlorethan, Decalin, Tetralin, Benzol, Toluol, Xylol und Mesitylen genannt. Zweckmäßigerweise verwendet man sowohl bei der Oxidation als auch bei der Cyclisierung das gleiche Lösungsmittel.

Nach beendeter Oxidation wird die organische von der wäßrigen Phase getrennt und ohne weitere Aufarbeitung direkt zur Cyclisierung eingesetzt, welche wie beschrieben ausgeführt wird. Das im Zuge der Cyclisierung anfallende azeotrope Destillat wird vom darin enthaltenen Wasser befreit und zur Isolierung des Formylcyclopropans zweckmäßig bei Atmosphärendruck oder unter vermindertem Druck fraktionierend destilliert. Auf diese Weise wird Formylcyclopropan in guten Ausbeuten erhalten.

Das erfindungsgemäße Verfahren ermöglicht somit die kostengünstige Herstellung von Formylcyclopropan aus einfachen Ausgangsverbindungen im industriellen Maßstab und ist dabei frei von den Mängeln der bisherigen Verfahrensweisen. Besonders hervorzuheben ist auch die leichte Übertragbarkeit des vorliegenden Verfahrens in die kontinuierliche Verfahrensweise, beispielsweise indem man die beiden Reaktionen in Rohrreaktoren in Kombination mit einem Phasenabscheider durchführt.

Die Ausgangsverbindung 4-Chlorbutanol ist ein Handelsprodukt, kann aber auch leicht durch Etherspaltung von Tetrahydrofuran mit Salzsäure hergestellt werden (s. hierzu: Org.Synth.Coll. Vol.II, 571 (1943)).

Beispiel 1

424 g (4,0 mol) Natriumcarbonat wurden in 1,5 l Wasser zusammen mit 29,6 g (0,1 mol) Tetrabutylammoniumchlorid und 200 ml Tetralin vorgelegt und auf 104°C erhitzt. Zu der intensiv gerührten Mischung wurden bei 104 bis 108°C 213 g (2,0 mol) 4-Chlorbutanal - gelöst in 200 ml Tetralin - getropft. Das Formylcyclopropan wurde, wie es sich bildete, als azeotropes Gemisch zusammen mit Wasser, Tetralin und wenig Dichlormethan und Tetrahydrofuran (Verunreinigungen aus der 4-Chlorbutanal-Herstellung) aus der Reaktionsmischung abdestilliert. Nach beendeter 4-Chlorbutanal-Zugabe wurde noch 15 min. lang weiter destilliert.

Das Destillat wurde von Wasser befreit und das Formylcyclopropan durch fraktionierte Destillation isoliert (Sdp: 94-97°C). Ausbeute: 71 %

Beispiel 2

38,8 g (0,25 mol) Triacetonamin wurden in 250 ml Dichlormethan gelöst. Diese Lösung wurde zur Erzeugung des Nitroxyl-Radikals tropfenweise und unter Kühlung bei Raumtemperatur mit einer Lösung von 0,52 mol m-Chlorperoxybenzoesäure in 500 ml Dichlormethan versetzt und nach der Zugabe noch eine halbe Stunde nachgerührt.

Zu dieser Lösung wurden anschließend bei Raumtemperatur weitere 250 ml Dichlormethan, 1000 ml Wasser, 542,5 g (5,0 mol) 4-Chlor-butanol, 29,8 g (0,25 mol) Kaliumbromid und 89,0 g (0,5 mol) Dinatriumhydrogenphosphat-Dihydrat gegeben. Dann wurde der pH-Wert der wäßrigen Phase durch Zugabe von Natronlauge auf 6,7 eingestellt.

Danach wurden unter intensivem Rühren, ebenfalls bei Raumtemperatur, 2660,7 g (5,0 mol) einer 14 Gew.-%igen wäßrigen Natriumhypochloritlösung langsam zugetropft. Nach der Zugabe wurde noch 15 min bei Raumtemperatur gerührt. Die das 4-Chlorbutanal enthaltende organische Phase wurde abgetrennt, die wäßrige Phase mit Dichlormethan extrahiert und die vereinigten organischen Phasen zu einer auf 110°C aufgeheizten Mischung - bestehend aus einer Lösung von 533 g Natriumhydroxid in 1750 ml Wasser, 40,0 g Methyltrioctylammoniumchlorid und 500 ml Tetralin - langsam zugetropft, wobei die Reaktionsmischung kräftig gerührt wurde.

Noch während der Zugabe wurde ein azeotropes Gemisch abdestilliert, das aus den Komponenten Dichlormethan, Tetrahydrofuran (entstanden durch Cyclisierung von nicht umgesetzten 4-Chlor-1-butanol), Wasser, Tetralin und Formylcyclopropan bestand. Während des Zutropfens der 4-Chlorbutanallösung wurden zusätzlich 750 ml Wasser zur Reaktionsmischung hinzugefügt, um das im Zuge der azeotropen Destillation abdestillierte Wasser zu ersetzen. Nach beendeter Zugabe und nachdem die Übergangstemperatur des azeotropen Gemisches 100°C erreicht hatte, wurde noch 15 min weiter destilliert. Das erhaltene azeotrope Destillat wurde vom Wasser befreit, und zur Isolierung des Formylcyclopropans fraktionierend destilliert.
Ausbeute (bez. auf 4-Chlorbutanol): 65 %

Beispiel 3

23,3 g (0,15 mol) Triacetonamin wurden in 150 ml Tetralin gelöst. Unter Kühlung wurde diese Lösung mit einer Lösung von 0,3 mol m-Chlorperoxybenzoesäure in 450 ml Tetralin versetzt und nach der Zugabe noch eine halbe Stunde nachgerührt.

Zu dieser Lösung wurden anschließend bei Raumtemperatur 600 ml Wasser, 325,5 g (3,0 mol) 4-Chlorbutanol, 17,9 g (0,15 mol) Kaliumbromid und 53,4 g (0,3 mol) Dinatriumhydrogenphosphat-Dihydrat gegeben. Durch Zugabe von Natronlauge wurde der pH-Wert der wäßrigen Phase auf 7,0 eingestellt.

Danach wurden unter intensivem Rühren 1650 g (3,1 mol) einer 14 Gew.%igen wäßrigen Natriumhypochloritlösung langsam zugetropft. Nach der Zugabe wurde noch für weitere 15 min bei Raumtemperatur gerührt. Die das 4-Chlorbutanal enthaltende organische Phase wurde abgetrennt, die wäßrige Phase zweimal mit Tetralin extrahiert und die vereinigten organischen Phasen zu einer auf 106°C aufgeheizten Mischung - bestehend aus 636 g (6,0 mol) Natriumcarbonat, 3 l Wasser, 60,6 g (0,15 mol) Methyltrioctylammoniumchlorid und 400 ml Tetralin - langsam zugetropft, wobei die Reaktionsmischung kräftig gerührt wurde. Noch während der Zugabe wurde ein azeotropes Gemisch abdestilliert, das aus den Komponenten Formylcyclopropan, Wasser, Tetralin und Tetrahydrofuran bestand. Während des Zutropfens der 4-Chlorbutanallösung wurden zusätzlich 750 ml Wasser zur Reaktionsmischung hinzugefügt. Nach beendeter Zugabe wurde noch 15 min weiter destilliert. Das erhaltene Destillat wurde vom Wasser befreit und das Formylcyclopropan durch fraktionierte Destillation isoliert.
Ausbeute (bez. auf 4-Chlorbutanol): 56 %

Beispiel 4

Eine Mischung aus 213 g (2,0 mol) 4-Chlorbutanal und 200 ml Tetralin wurde innerhalb 90 Minuten zu einem intensiv gerührten, siedenden Gemisch, bestehend aus 254 g (2,4 mol) Natriumcarbonat gelöst in 1500 ml Wasser und 200 ml Tetralin, getropft. Bereits während dieser Zugabe wurde bei Atmosphärendruck ein azeotropes Gemisch aus Formylcyclopropan, unverbrauchtem 4-Chlorbutanal, Wasser und Tetralin abdestilliert.

Die wäßrige Phase des Destillats wurde in einem Phasenabscheider von der organischen Phase abgetrennt und nochmals extrahiert. Anschließend wurden die vereinigten, organischen Phasen über Natriumsulfat getrocknet und dann fraktionierend destilliert.
Ausbeute: 71 %

## Patentansprüche

1. Verfahren zur Herstellung von Formylcyclopropan (I)

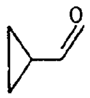

I

durch Cyclisierung von 4-Chlorbutanal mittels einer Mineralbase bei Temperaturen von 50 bis 150°C, dadurch gekennzeichnet, daß man die Umsetzung mittels einer wäßrigen Mineralbase in einem flüssigen Zweiphasensystem, bestehend aus einer wäßrigen und einer nicht mit Wasser mischbaren, organischen Phase, vornimmt und das Formylcyclopropan laufend aus dem Reaktionsgemisch entfernt.

2. Verfahren nach Anspruch 1, dadurch gekennzeichnet, daß man die Umsetzung in Gegenwart eines Phasentransferkatalysators vornimmt

3. Ausgestaltung des Verfahrens gemäß Anspruch 1, dadurch gekennzeichnet, daß man die Ausgangsverbindung 4-Chlorbutanal durch Oxidation von 4-Chlorbutanol in einem Zweiphasensystem herstellt, wobei als Oxidationsmittel ein System verwendet wird, das aus einer wäßrigen Natriumhypochloritlösung und einer Verbindung der allgemeinen Formel II

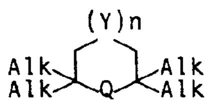

II

besteht, in der Alk gleiche oder verschiedene C1-C4-Alkylgruppen bezeichnet, Q für eine der Gruppierungen

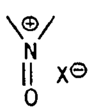 oder 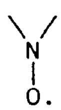 oder 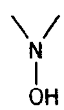

steht, wobei $X^{\ominus}$ ein Anion bedeutet, n 0 oder 1 ist und Y für Sauerstoff, die Carbonylgruppe oder einen Rest

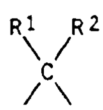

steht, in welchem $R^1$ und $R^2$ Wasserstoff, eine Hydroxylgruppe oder C-organische oder O-organische Reste bedeuten, die auch miteinander zu einem fünf- oder sechsgliedrigen Ring verbunden sein können, und die anschließende Cyclisierung gemäß dem Verfahren nach Anspruch 1 durchführt, ohne das 4-Chlorbutanal vorher zu isolieren.

4. Verfahren nach Anspruch 1, dadurch gekennzeichnet, daß man die Cyclisierung bei Temperaturen von 100 bis 120° vornimmt.

5. Verfahren nach Anspruch 3, dadurch gekennzeichnet, daß man die Oxidation in Gegenwart katalytischer Mengen Kaliumbromid durchführt

6. Verfahren nach Anspruch 3, dadurch gekennzeichnet, daß man es kontinuierlich in einem Rohrreaktor durchführt.

## Claims

1. A process for the preparation of formylcyclopropane (I)

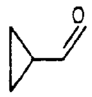

I

by cyclization of 4-chlorobutanal by means of a mineral base at 50-150°C, wherein the reaction is carried out by means of an aqueous mineral base in a liquid two-phase system consisting of an aqueous phase and a water-immiscible organic phase, and the formylcyclopropane is removed continuously from the reaction mixture.

2. A process as claimed in claim 1, wherein the reaction is carried out in the presence of a phase-transfer catalyst.

3. An embodiment of the process as claimed in claim 1, wherein the starting compound 4-chlorobutanal is prepared by oxidation of 4-chlorobutanol in a two-phase system, the oxidizing agent used being a system which consists of an aqueous sodium hypochlorite solution and a compound of the formula II

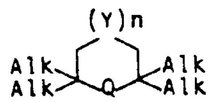

II

where the radicals Alk are identical or different $C_1$-$C_4$-alkyl groups, Q is one of the groups

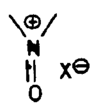 or 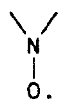 or 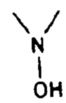

$X^{\ominus}$ is an anion, n is 0 or 1 and Y is oxygen, the carbonyl group or a radical

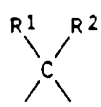

and $R^1$and $R^2$ are each hydrogen, hydroxyl or a C-organic or O-organic radical which may furthermore be bonded to one another to form a 5-membered or 6-membered ring, and the subsequent cyclization according to the process as claimed in claim 1 is carried out without isolating the 4-chlorobutanal beforehand.

4. A process as claimed in claim 1, wherein the cyclization is carried out at from 100 to 120°C.

5. A process as claimed in claim 3, wherein the oxidation is carried out in the presence of a catalytic amount of potassium bromide.

6. A process as claimed in claim 3, which is carried out continuously in a tube reactor.

## Revendications

1. Procédé de préparation de formylcyclopropane (I)

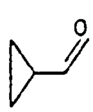

I

par cyclisation de 4-chlorobutanal au moyen d'une base minérale à des températures de 50 à 150°C, caractérisé en ce qu'on mène la réaction au moyen d'une base minérale aqueuse dans un système liquide à deux phases, se composant d'une phase aqueuse et d'une phase organique non miscible à l'eau, et on enlève en permanence du mélange réactionnel le formylcyclopropane.

2. Procédé selon la revendication 1, carartérisé en ce qu'on mène la réaction en présence d'un catalyseur

de transfert de phase.

3. Mode d'exécution du procédé selon la revendication 1, caractérisé en ce qu'on prépare le composé de départ 4-chlorobutanal par oxydation de 4-chlorobutanol dans un système à deux phases, en utilisant comme agent d'oxydation un système qui se compose d'une solution aqueuse d'hypochlorite de sodium et d'un composé de formule générale II

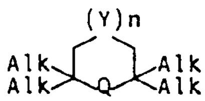

II

dans laquelle Alk représente des groupements alkyle en $C_1$-$C_4$ identiques ou différents, Q est mis pour l'un des groupements

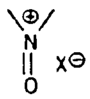

ou

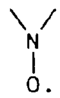

ou

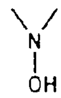

$X^{\ominus}$ représentant un anion, n étant mis pour 0 ou 1 et Y pour un atome d'oxygène, pour le groupement carbonyle ou pour un reste

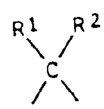

où $R^1$ et $R^2$ représentent chacun un atome d'hydrogène, un groupement hydroxy ou des restes C-organiques ou O-organiques qui peuvent être également reliés entre eux en un noyau penta- ou hexagonal, et on effectue ensuite la cyclisation par le procédé selon la revendication 1, sans isoler préalablement le 4-chlorobutanal.

4. Procédé selon la revendication 1, caractérisé en ce qu'on procède à la cyclisation à des températures de 100 à 120°C.

5. Procédé selon la revendication 3, caractérisé en ce qu'on conduit l'oxydation en présence de quantités catalytiques de bromure de potassium.

6. Procédé selon la revendication 3, carartérisé en ce qu'on le conduit de façon continue dans un réacteur tubulaire.